(19) Europäisches Patentamt

European Patent Office

Office européen des brevets

(11) Publication number: **0 080 632**
**B1**

(12) **EUROPEAN PATENT SPECIFICATION**

(45) Date of publication of patent specification: **19.02.86**

(51) Int. Cl.⁴: **G 01 N 27/22**, G 01 R 27/26

(21) Application number: **82110482.5**

(22) Date of filing: **12.11.82**

(54) **Oil deterioration detector.**

(30) Priority: **17.11.81 JP 182996/81**

(43) Date of publication of application:
**08.06.83 Bulletin 83/23**

(45) Publication of the grant of the patent:
**19.02.86 Bulletin 86/08**

(84) Designated Contracting States:
**DE FR GB**

(56) References cited:
EP-A-0 001 919
DE-A-2 120 744
US-A-3 067 385

JOURNAL OF PHYSICS. E. SCIENTIFIC
INTRUMENTS, vol. 12, no. 9, 1979 J. BLOM
"Measurement of dielectric relaxation of
conducting solutions at low frequencies" pages
889-893

(73) Proprietor: **NISSAN MOTOR CO., LTD.**
No.2, Takara-cho, Kanagawa-ku
Yokohama-shi Kanagawa-ken 221 (JP)

(72) Inventor: **Yasuhara, Seishi**
9-6, Highland 4-chome
Yokosuka-shi Kanagawa-ken (JP)
Inventor: **Kobayashi, Hiroshi**
15-B-412, Ooka 4-chome
Minami-ku Yokohama-shi Kanagawa-ken (JP)
Inventor: **Kita, Toru**
23-18-314, Highland 1-chome
Yokosuka-shi Kanagawa-ken (JP)
Inventor: **Saito, Hideyuki**
16-6, Nishigotanda 4-chome
Shinagawa-ku Tokyo (JP)

(74) Representative: **Patentanwälte TER MEER -
MÜLLER - STEINMEISTER**
Mauerkircherstrasse 45
D-8000 München 80 (DE)

## Description

The invention relates to an apparatus for detecting the degree of deterioration of lubricating oil according to the preamble of claim 1.

If lubricating oil is used for a longer period of time in internal combustion engines, the lubricating oil gets deteriorated to have its lubricating ability reduced due to the entrance of contaminants such as soot. It is desirable to know the state of the lubricating oil so that the oil can be changed at a proper time.

The document EP—A—19 19 describes an apparatus for analysing the amount of contaminants in a fluid by determining the dielectric constant of the fluid. This known apparatus includes an AC voltage source and a sensor capacitor including a pair of spaced apart electrodes immersed in the fluid, and a circuit connecting the sensor capacitor to said AC voltage source for delivering an output corresponding to the dielectric constant of the fluid to an indicator which may include an AC voltmeter.

However, this known apparatus is designated for laboratory investigations and is not suited for monitoring the deterioration of lubricating oil used in an internal combustion engine.

Lubricating oil is subject to temperature changes which result in changes of the dielectric constant. In order to compensate for temperature changes of the dielectric constant, the document US—A—3 067 385 discloses a lubricating oil monitoring apparatus, wherein the electrodes of a sensor capacitor are shifted with respect to each other in response to the temperature of the lubricating oil.

On the other hand, it is known to determine the degree of deterioration of lubricating oil used in internal combustion engines by measuring the conductivity of the lubricating oil by sensing current flow between a pair of electrodes immersed in the lubricating oil within an oil pan. However, the conductivity of lubricating oil varies greatly with its temperature and brand to such an extent as to completely invalidate the expected relationship between the measurement of the conductivity of the lubricating oil and the degree of deterioration of lubricating oil. This is true particularly for Diesel engines where a great amount of soot enters the lubricating oil.

The present invention aims at providing an apparatus which can detect the degree of deterioration of lubricating oil used in internal combustion engines with greater accuracy independently of lubricating oil temperature and brand.

In accordance with the present invention, there is provided an apparatus for detecting the degree of deterioration of lubricating oil used in an internal combustion engine, comprising an AC voltage source for generating an AC voltage of a predetermined frequency ranging from 50 KHz to 500 KHz, a sensor including a pair of spaced apart electrodes immersed in lubricating oil to form a sensor capacitor whose capacity varies as a function of the dielectric constant of the lubricating oil and a circuit connecting said sensor capacitor to said AC voltage source for generating an output corresponding to the lubricating oil dielectric constant; and an indicator connector to said sensor for indicating an oil deterioration degree corresponding to the output of said sensor, characterized in that said indicator includes a gauge for providing an oil deterioration degree indication corresponding to the output of said sensor and means for holding said gauge out of operation until the temperature of an internal combustion engine in which the lubricating oil is used reaches a predetermined value.

The details as well as other features and advantages of this invention are set forth below and are shown in the accompanying drawings, in which:

Fig. 1 is a block diagram showing one embodiment of an oil deterioration detector made in accordance with the present invention;

Fig. 2 is a graph showing variations in the impedance and capacity of the sensor capacitor with the lubricating oil being getting deteriorated;

Figs. 3A, 3B and 3C show two different forms of the sensor capacitor;

Figs. 4A and 4B are schematic views showing a lubrication system in which the sensor capacitor is placed;

Fig. 5 is a schematic view showing a lubrication system oil passage in which the sensor capacitor is placed;

Fig. 6 shows an oil chamber formed in the lubrication system for reception of the sensor capacitor;

Fig. 7 is an elevational view showing a gauge included in the oil deterioration indicator of Fig. 1;

Fig. 8 is a circuit diagram of the oil deterioration indicator;

Fig. 9 is a comparative graph showing impedance versus AC voltage frequency curves obtained on new and used lubricating oil at low and high temperatures; and

Fig. 10 is a comparative graph showing specified inductive capacity versus AC voltage frequency curves obtained in new and used lubricating oil at low and high temperatures.

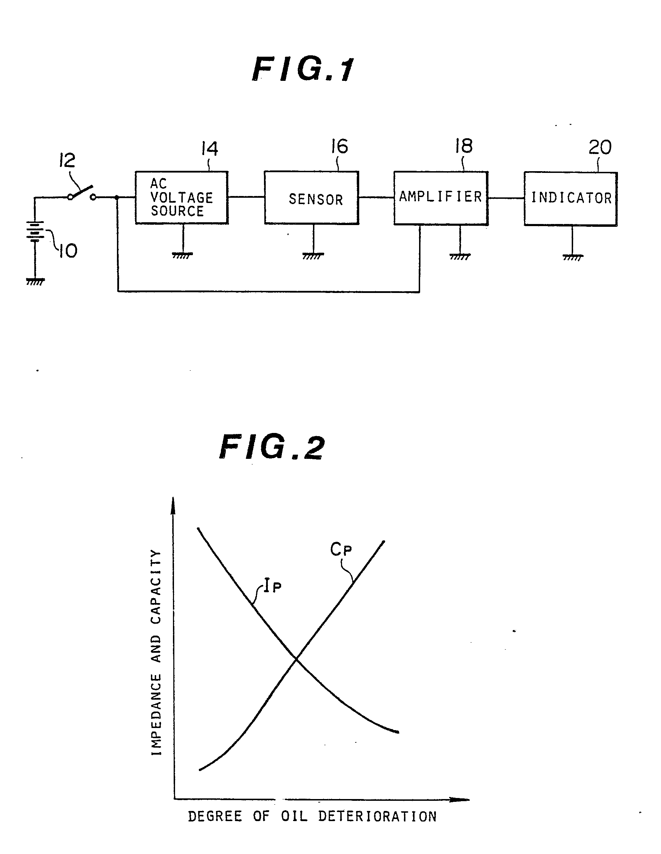

Referring now to Fig. 1 of the drawings, there is shown one embodiment of an oil deterioration detecting apparatus made in accordance with the present invention. A DC power source 10 is connected through a main switch 12 to an AC voltage generator 14. The AC voltage generator 14 comprises a CR oscillator or a crystal oscillator for generating an AC voltage of a constant frequency when the main switch 12 is closed. The frequency of the AC voltage is selected at a value within a range of 50 KHz to 500 KHz as will be described in detail. The AC voltage is applied to a sensor 16 which generates an AC voltage corresponding to the degree of deterioration of an engine oil used in an internal combustion engine. The output of the sensor 16 is coupled through an amplifier 18 to an oil deterioration indicator 20 for providing an indication of the sensed degree of oil deterioration.

The sensor 16 includes a sensor capacitor which has a pair of spaced apart electrodes dipped under the lubricating oil so that the sensor capacitor has a capacity more than 400 pF to avoid an adverse influence of floating capacity. For example, the electrodes may be taken in the form of about 200 cm² area plates spaced apart a distance of about 1 mm from each other. The impedance and capacity of the sensor capacitor vary as a function of the dielectric constant or permittivity of the lubricating oil, as shown in Fig. 2. As the lubricating oil gets more deteriorated to increase its dielectric constant, the sensor capacitor impedance decreases as illustrated by impedance versus oil deterioration curve IP and the sensor capacitor capacity increases as illustrated by capacity versus oil deterioration curve CP. The sensor also includes a circuit which connects the sensor capacitor to the AC voltage generator for generating a sensor output corresponding to the dielectric constant of the lubricating oil. The circuit may be taken in the form of a capacitor having a fixed capacity, in which case the capacitor is connected in series with the sensor capacitor to form a voltage divider which divides the output voltage of the AC voltage generator 14 at a ratio determined by the dielectric constant of the lubricating oil so that the AC voltage across the sensor capacitor is substantially proportional to the engine oil dielectric constant.

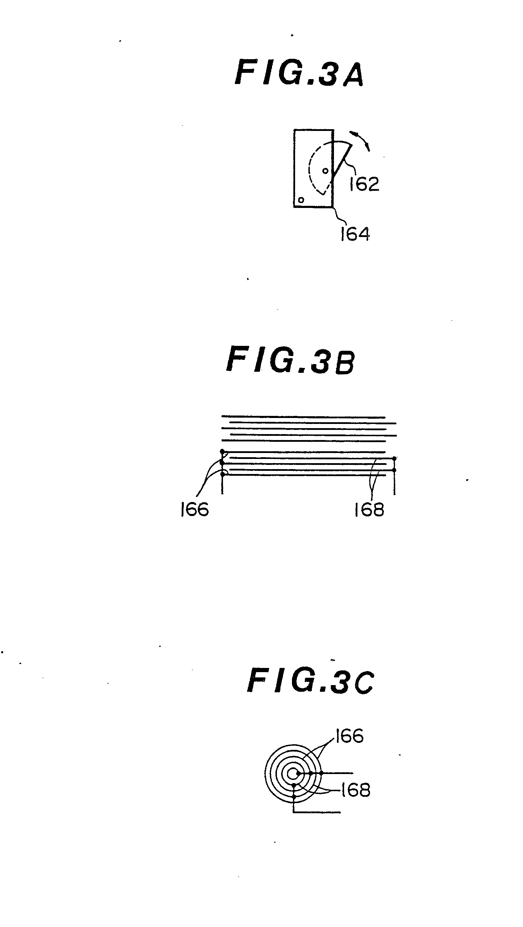

The sensor capacitor may be a variable capacitor, as shown in Fig. 3A, which comprises one set of parallel plates 162 moving in relation to another set of parallel plates 164 to continuously vary the value of capacity. Alternatively, the sensor capacitor may have one set of concentrically arranged cylindrical plates 166 placed in relation to another set of concentrically arranged cylindrical plates 168 as shown in Figs. 3B and 3C. Such capacitor arrangements are effective to reduce the sensor size and facilitate the lubricating oil to shift through the sensor capacitor. The sensor capacitor may be a printed electrode plate having a desired electrode figure printed on a top plate.

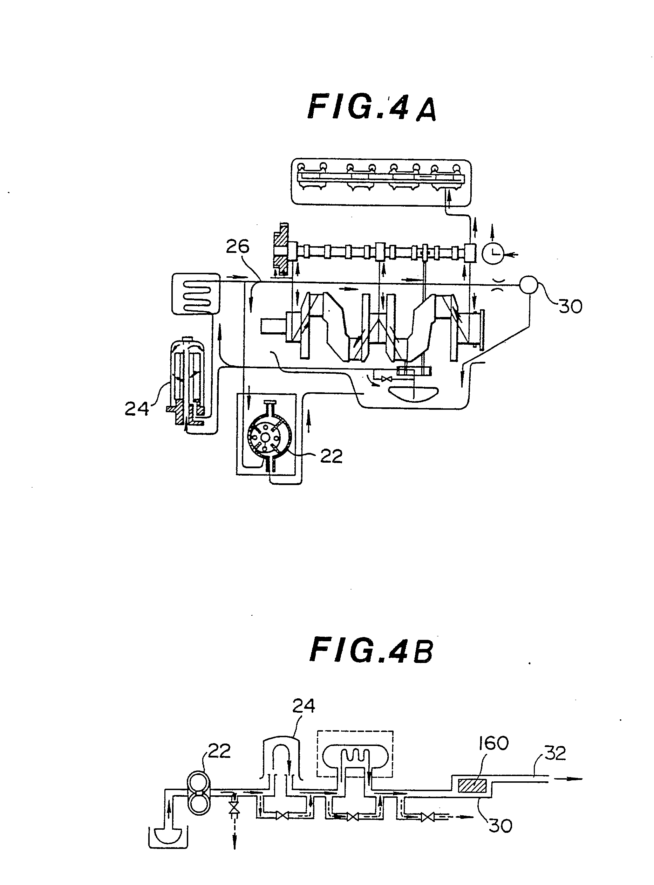

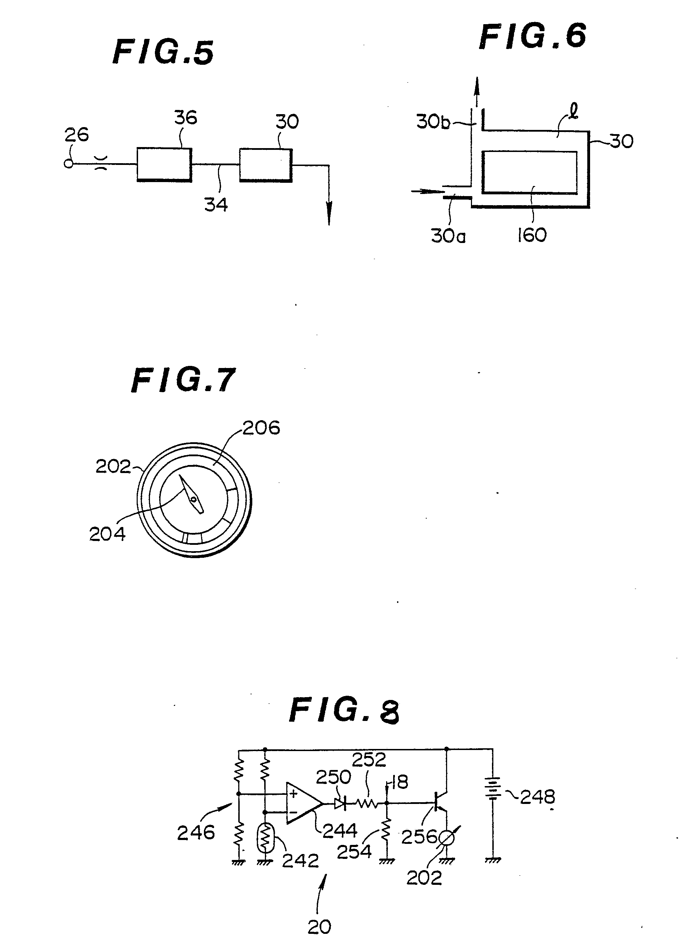

Referring to Figs. 4A and 4B, there is illustrated a lubrication system which includes a pump 22 for supplying engine oil under pressure through an oil filter 24 to a main gallery 26. The sensor capacitor 160 is placed in an oil chamber 30 formed in an oil passage 28 downstream of the main gallery 26 or in an oil passage 32 downstream of the oil filter 24. The oil chamber may be formed in other suitable position as long as there is a minimum amount of contaminants and the engine oil flows at a relatively high rate in order to keep the sensor capacitor free from any deposite on its electrode surfaces. This is effective to keep at a minimum changes in the characteristics of the sensor capacitor with time. Alternatively, the oil chamber 30 may be formed in a return passage 34 leading to an oil pan with another oil filter 36 being positioned in the return passage 34 upstream of the oil chamber 30 as shown in Fig. 5.

Referring to Fig. 6, the oil chamber 30, which contains the sensor capacitor 160, has an inlet 30a formed near its bottom and an outlet 30b formed in its upper wall so that air cannot be stored in the oil chamber 30. Preferably, the sensor capacitor 160 is placed in the oil chamber 30 to provide a space of l between its electrodes and the oil chamber upper wall with its electrodes extending vertically toward the oil chamber upper wall to avoid existence of air bubbles between the sensor capacitor electrodes to change the capacity of the sensor capacitor. The sensor capacitor electrodes are coated on their surfaces with a corrosion protective material such as fluorocarbon polymers to protect them against acids and bases included in the lubricating oil. This is also effective to prevent DC conduction from occurring due to entrance of contaminants between the sensor capacitor electrodes.

The output of the sensor 16 is applied to an amplifier 18 which amplifies the AC voltage across the sensor capacitor and applies the amplified AC voltage to the oil deterioration indicator 20. The amplifier 18 may have an additional function of converting the amplified AC voltage value into a corresponding DC voltage value.

Referring to Fig. 7, the oil deterioration indicator 20 includes a gauge 202 which has a pointer 204 and a scale with ranges classified by color.

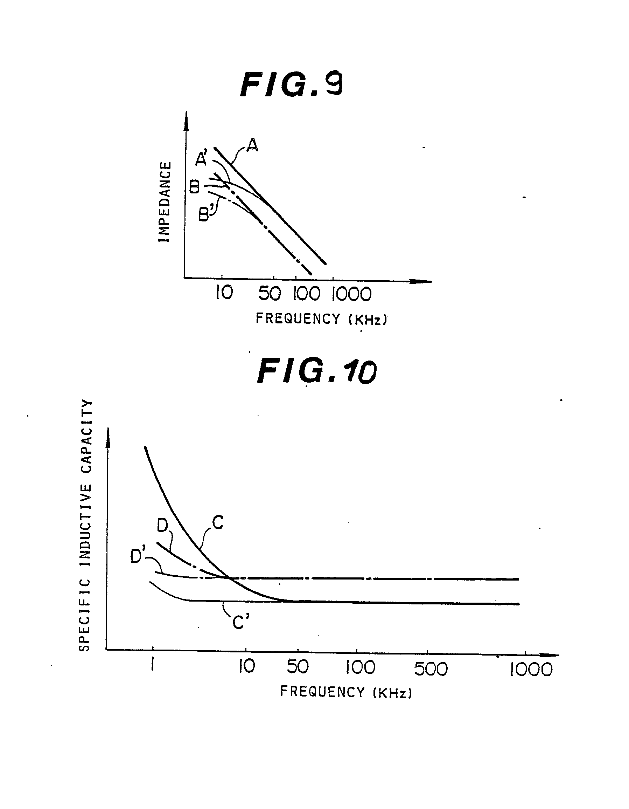

Fig. 9 shows comparative curves of impedance versus AC voltage frequency. The curve marked A is obtained on new engine oil at high temperature and the curve marked A' is obtained on new engine oil at low temperature. The curve marked B is obtained on used engine oil at high temperature and the curve marked B' is obtained on used engine oil at low temperature. From Fig. 9 it may be seen that a greater change is caused in the impedance of the sensor capacitor by engine temperature changes than is caused by oil deterioration when the frequency of the AC voltage applied to the sensor capacitor is below about 50 KHz. Comparative specific inductive capacity curves are given in Fig. 10. The curve marked C is obtained on new engine oil at high temperature and the curve marked C' is obtained on new engine oil at low temperature. The curve marked D is obtained on used engine oil at high temperature and the curve marked D' is obtained on used engine oil at low temperature. It may be seen from Fig. 10 that a greater change is caused in the specific inductive capacity of the engine oil by engine temperature changes than is caused by oil deterioration when the frequency of the AC voltage applied to the sensor capacitor is below about 50 KHz.

The frequency of the AC voltage applied from the AC voltage generator 14 to the sensor 16 is set at a value within a range of 50 KHz to 500 KHz. If the frequency is lower than this range, the oil deterioration measurement made in the sensor 16 is susceptible to influences from engine temperature changes, causing an improper indication of oil deterioration degree. If it is higher than the range, radio interference occurs in AM band. Preferably, the frequency is set at 100 KHz. Experiments show that any deviation which appears on the output of the sensor 16 due to use of lubrication oil of different brands can be ignored in practice.

Referring to Fig. 8, there is illustrated an oil deterioration indicator 20 wherein a circuit is associated with the gauge 202 for placing the gauge 202 in operation only when the engine temperature is above a predetermined value, for example, 80°C. This is effective to avoid adverse influence of engine temperature changes on the indication of the oil deterioration degree. The circuit comprises a thermistor 242 connected in an electrical circuit capable of producing a DC voltage having a variable level corresponding to the engine temperature represented by the engine coolant temperature. The output of the thermistor 242 is connected to the negative input terminal of an comparator 244 whose positive input terminal is connected to a reference voltage source 246 which is taken in the form of a voltage divider connector across a DC voltage source 248 for generating a reference voltage which represents the predetermined temperature value. The output of the comparator 244 is connected through a diode 250 and a resistor 252 to the base of a transistor 256 whose emitter is grounded through the gauge 202. The collector of the transistor 256 is connected to the positive terminal of the DC power source 248. The base of the transistor 256 is connected through a resistor 254 to ground and also to the output of the amplifier 18. When the engine coolant temperature reaches the predetermined value, the comparator 244 generates an high output which conducts the transistor 256 to place the gauge 202 in operation.

While the present invention has been described in conjunction with specific embodiments thereof, it is evident that many alternatives, modifications and variations will be apparent to those skilled in the art. Accordingly, it is intended to embrace all alternatives, modifications and variations that fall within the scope of the appended claims.

## Claims

1. An apparatus for detecting the degree of deterioration of lubricating oil used in an internal combustion engine, comprising

(a) an AC voltage source (14) for generating an AC voltage of a predetermined frequency ranging from 50 KHz to 500 KHz,

(b) a sensor (16) including a pair of spaced apart electrodes (162, 164; 166, 168) immersed in lubricating oil to form a sensor capacitor whose capacity varies as a function of the dielectric constant of the lubricating oil and a circuit connecting said sensor capacitor to said AC voltage source (14) for generating an output corresponding to the lubricating oil dielectric constant; and

(c) an indicator (20) connected to said sensor (16) for indicating an oil deterioration degree corresponding to the output of said sensor,

characterized in that said indicator (20) includes a gauge (202) for providing an oil deterioration degree indication corresponding to the output of said sensor (16) and means (242, 244, 256) for holding said gauge (202) out of operation until the temperature of an internal combustion engine in which the lubricating oil is used reaches a predetermined value.

2. Apparatus according to claim 1, characterized in that said AC voltage source (14) generates an AC voltage of a constant frequency of 100 KHz.

3. Apparatus according to claim 1 or 2, characterized in that said sensor capacitor is a variable capacitor including one set of parallel metal plates (162) moving in relation to another set of parallel plates (164) to continuously vary the capacity.

4. Apparatus according to any of the claims 1 to 3, characterized in that said sensor capacitor includes one set of concentrically arranged cylindrical metal plates (166) arranged in relation to another set of concentrically arranged cylindrical metal plates (168).

5. Apparatus according to any of the above claims, characterized in that said circuit includes a capacitor of a fixed capacity, said capacitor being connected in series with said sensor capacitor to form a voltage divider connected across said AC voltage source (14) so that an AC voltage corresponding to the lubricating oil dielectric constant is developed across said sensor capacitor.

## Patentansprüche

1. Vorrichtung zur Abtastung des Grades der Qualitätsminderung von Schmieröl in einer Brennkraftmaschine, mit

(a) einer Wechselspannungsquelle (14) zur Erzeugung einer Wechselspannung mit einer vorgegebenen Frequenz im Bereich von 50 KHz bis 500 KHz,

(b) einem Sensor (16) mit zwei in Abstand angeordneten, in Schmieröl eingetauchten Elektroden (163, 164; 166, 168) zur Bildung eines Meßkondensators, dessen Kapazität als Funktion der Dielektrizitätskonstanten des Schmieröls veränderlich ist, und einer den Meßkondensator mit der Wechselspannungsquelle (14) verbindenden Schaltung zur Erzeugung eines der Dielektrizitätskonstanten des Schmieröls entsprechenden Ausgangssignals; und

(c) einem mit dem Sensor (16) verbundenen Anzeigeinstrument (20) zur Anzeige des Grades der Qualitätsminderung des Öls entsprechend dem Ausgangssignal des Sensors,

dadurch gekennzeichnet, daß das Anzeigeinstrument (20) eine Meßuhr (202) zur Erzeugung einer dem Grad der Qualitätsminderung des Öls entsprechenden Anzeige anhand des Ausgangssignals des Sensors (16) und eine Einrichtung (242, 244, 256) zum Außerbetriebsetzen der Meßuhr (202) umfaßt, bis die Temperatur einer Brennkraftmaschine, in der das Schmieröl verwendet wird, einen vorgegebenen Wert erreicht.

2. Vorrichtung nach Anspruch 1, dadurch gekennzeichnet, daß die Wechselspannungsquelle (14) eine Wechselspannung mit einer konstanten Frequenz von 100 KHz erzeugt.

3. Vorrichtung nach Anspruch 1 oder 2, dadurch gekennzeichnet, daß der Meßkondensator ein einstellbarer Kondensator ist und einen Satz paralleler Metallplatten (162) umfaßt, die zur kontinuierlichen Veränderung der Kapazität in Bezug auf einen anderen Satz paralleler Platten (164) beweglich sind.

4. Vorrichtung nach einem der Ansprüche 1 bis 3, dadurch gekennzeichnet daß der Meßkondensator einen Satz konzentrisch angeordneter zylindrischer Metallplatten (166) umfaßt, die einem weiteren Satz konzentrisch angeordneter zylindrischer Metallplatten (168) zugeordnet sind.

5. Vorrichten nach einem der obigen Ansprüche, dadurch gekennzeichnet, daß die Schaltung einen Kondensator mit fester Kapazität umfaßt, der mit dem Meßkondensator zur Bildung eines parallel zu der Wechselspannungsquelle (14) geschalteten Spannungsteilers in Serie geschaltet ist, so daß eine der Dielektrizitätskonstanten des Schmieröls entsprechende Wechselspannung über dem Meßkondensator abfällt.

**Revendications**

1. Appareil de détection du degré de détérioration d'une huile de lubrification utilisée dans un moteur à combustion interne, comprenant

(a) une source (14) de tension alternative pour la génération d'une tension alternative d'une fréquence prédéterminée comprise dans une plage de 50 kHz à 500 kHz,

(b) un détecteur (16) comportant une paire d'électrodes (162, 164; 166, 168) espacées l'une de l'autre et plongées dans l'huile de lubrification de manière à former un condensateur détecteur dont la capacité varie en fonction de la constante diélectrique de l'huile de lubrification, et un circuit reliant ledit condensateur détecteur à ladite source (14) de tension alternative pour fournir une tension de sortie correspondant à la constante diélectrique de l'huile de lubrification; et

(c) un dispositif d'indication (20) relié audit détecteur (16) pour indiquer le degré de détérioration de l'huile correspondant à la tension de sortie dudit détecteur,

caractérisé en ce que ledit dispositif d'indication (20) comprend un indicateur (202) fournissant une indication du degré de détérioration de l'huile correspondant à la tensions de sortie dudit détecteur (16), et des moyens (242, 244, 256) pour maintenir ledit indicateur (202) en état de non fonctionnement jusqu'à ce que la température du moteur à combustion interne, dans lequel est utilisée l'huile de lubrification, atteigne une valeur prédéterminée.

2. Appareil suivant la revendication 1, caractérisé en ce que ladite source (14) de tension alternative fournit une tension alternative d'une fréquence constante de 100 kHz.

3. Appareil suivant la revendication 1 ou 2, caractérisé en ce que ledit condensateur détecteur est un condensateur variable comprenant un jeu de plaques métalliques parallèles (162) mobiles par rapport à un autre jeu de plaques parallèles (164) de manière à varier en continu la capacité du condensateur.

4. Appareil suivant l'une quelconque des revendications 1 à 3, caractérisé en ce que ledit condensateur détecteur comprend un jeu de plaques métalliques cylindriques (166) disposées concentriquement et en relation avec un autre jeu de plaques métalliques cylindriques (168) disposées concentriquement.

5. Appareil suivant l'une quelconque des revendications ci-dessus, caractérisé en ce que ledit circuit comprend un condensateur de capacité fixe, ledit condensateur étant relié en série avec ledit condensateur détecteur pour former un diviseur de tension relié aux bornes de ladite source (14) de tension alternative afin qu'une tension alternative correspondant à la constante diélectrique de l'huile de lubrification apparaisse aux bornes dudit condensateur détecteur.

# FIG.1

# FIG.2

DEGREE OF OIL DETERIORATION

# FIG.3A

162

164

# FIG.3B

166                    168

# FIG.3C

166

168

# FIG.4A

# FIG.4B

## FIG.5

## FIG.6

## FIG.7

## FIG.8

0 080 632

# FIG.9

IMPEDANCE

FREQUENCY (KHz)

# FIG.10

SPECIFIC INDUCTIVE CAPACITY

FREQUENCY (KHz)